(19) Europäisches Patentamt
European Patent Office
Office européen des brevets

(11) Veröffentlichungsnummer: **0 017 039**
**B1**

(12) # EUROPÄISCHE PATENTSCHRIFT

(45) Veröffentlichungstag der Patentschrift: **12.01.83**

(51) Int. Cl.³: **C 07 D 277/16**

(21) Anmeldenummer: **80101289.9**

(22) Anmeldetag: **13.03.80**

(54) **Verfahren zur Herstellung von Thiazolidin-2-thionen.**

(30) Priorität: **24.03.79 DE 2911662**

(43) Veröffentlichungstag der Anmeldung: **15.10.80 Patentblatt 80/21**

(45) Bekanntmachung des Hinweises auf die Patenterteilung: **12.01.83 Patentblatt 83/2**

(84) Benannte Vertragsstaaten: **BE DE FR GB IT NL**

(56) Entgegenhaltungen:

**US-A-3 215 703**
**US-A-3 215 704**
**JOURNAL OF THE CHEMICAL SOCIETY, 1949, Part II, Seiten 786-789, London, GB,**
**J.W. BATTY et al.: «Acetylene reactions. Part III. Reaction of aminobutynes with carbon disulphide»**

(73) Patentinhaber: **BAYER AG, Zentralbereich Patente, Marken und Lizenzen, D-5090 Leverkusen 1, Bayerwerk (DE)**

(72) Erfinder: **Schubart, Rüdiger, Dr., An der Engelsfuhr 27, D-5060 Bergisch-Gladbach 2 (DE)**

**EP 0 017 039 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Thiazolidin-2-thionen

Die Erfindung betrifft ein Verfahren zur Herstellung von Thiazolidin-2-thionen, die durch Umsetzung von Aminoalkoholen mit Schwefelkohlenstoff erhalten werden.

Die US-PS 3 215 704 erwähnt als Stand der Technik obige Umsetzung und führt dazu aus, dass man in niedrigen Ausbeuten stark verunreinigte Produkte erhält. Dieser Nachteil — so wird geschildert — kann dadurch behoben werden, dass man die Reaktion in zwei Verfahrensstufen aufteilt. In der ersten Stufe wird zunächst der Aminoalkohol bei einer Temperatur von unter 100°C mit einem Teil der erforderlichen Menge an Schwefelkohlenstoff zum Dithiocarbamat umgesetzt und dieses dann bei einer Temperatur von über 110°C in einer zweiten Stufe mit einer weiteren Menge an Schwefelkohlenstoff zur Reaktion gebracht. Die erhaltenen Produkte können direkt ohne weitere Reinigung als Vulkanisationsverzögerer eingesetzt werden.

Es zeigt sich jedoch, dass diese Produkte immer noch einen unangenehmen Geruch besitzen, was darauf zurückgeführt werden kann, dass immer noch in erheblichem Masse Verunreinigungen anwesend sind. Dies geht letztlich auch daraus hervor, dass die gemäss den Beispielen erhaltenen Produkte niedriger schmelzen, als der theoretische Wert es vorsieht.

Auch die US-PS 3 215 703 beschreibt ein zweistufiges Herstellungsverfahren für Thiazolidin-2-thione, bei dem in längerer Reaktionszeit in geringer Reinheit die gewünschten Endprodukte in niedriger Ausbeute erhalten werden.

Aufgabe der vorliegenden Erfindung ist es, ein Einstufenverfahren zur Herstellung von Thiazolidin-2-thionen bereitzustellen, welches in hoher Ausbeute das gewünschte Endprodukt liefert.

Gegenstand der Erfindung ist somit ein Verfahren zur Herstellung von Thiazolidin-2-thionen der allgemeinen Formeln 1 und 2

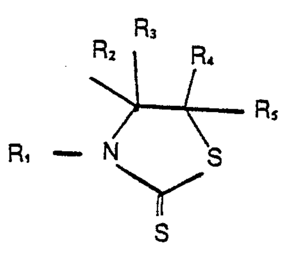

(1)

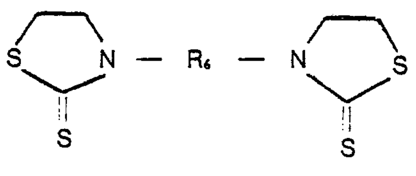

(2)

in denen

$R_1$ Wasserstoff oder einen geradkettigen, verzweigten oder cyclischen Alkylrest oder Alkenylrest, der gegebenenfalls durch Heteroatome (O, S, N) unterbrochen sein kann oder einen Arylrest bedeutet, wobei die aufgeführten Reste gegebenenfalls auch durch Mono- oder Dialkylamino-, Alkoxy-, Alkylthio- und/oder Halogengruppen substituiert sein können und im Falle eines Arylrestes noch zusätzlich ein Alkylrest vorhanden sein kann,

$R_2$ und $R_3$ gleich oder verschieden Wasserstoff, einen Alkylrest oder Phenylrest bedeuten, wobei Alkylreste untereinander zu einem 5- oder 6gliedrigen Ring verbunden sein können,

$R_4$ und $R_5$ Wasserstoff, einen Alkyl- oder Vinylrest und

$R_6$ eine Alkylen- oder Phenylengruppe darstellt,

durch Reaktion von entsprechenden Aminoalkoholen mit Schwefelkohlenstoff, das dadurch gekennzeichnet ist, dass Schwefelkohlenstoff im molaren Mengenverhältnis zu Aminoalkohol von 4:1 bis 10:1 zugeführt und dass die Reaktion bei Temperaturen von 145-220°C und bei Normaldruck bis 100 bar in einstufiger Verfahrensweise durchgeführt wird.

Es konnte überraschenderweise festgestellt werden, dass die Reaktionsgeschwindigkeit der beiden Komponenten durch die Anwesenheit von mehr als der theoretisch zur Reaktion notwendigen Menge an Schwefelkohlenstoff erheblich gesteigert wird. Das molare Mengenverhältnis von Schwefelkohlenstoff zu Aminoalkohol beträgt bevorzugt 4-6:1. Die Reaktionstemperatur ist bevorzugt 150-200°C. Der bei der Umsetzung angewendete Druck kann in weiten Grenzen variieren. Er beträgt bevorzugt 20 bis 70 bar. Generell lässt sich sagen, dass man bei grossem Überschuss von Schwefelkohlenstoff bei Normaldruck arbeiten kann, während man bei einem geringeren Überschuss einen gewissen Druck anlegen muss. Um nach Beendigung der Reaktion nicht zuviel nicht umgesetzten Schwefelkohlenstoff aufarbeiten zu müssen, erscheint es empfehlenswert, in nicht zu grossem Überschuss an Schwefelkohlenstoff zu arbeiten.

Die Reaktion lässt sich diskontinuierlich oder kontinuierlich durchführen. Sie ist in relativ kurzer Zeit beendet. Aufgrund der kurzen Reaktionszeit erscheint es empfehlenswert, eine kontinuierliche Verfahrensweise durchzuführen.

Als Reaktor wird im Prinzip ein Gefäss eingesetzt, das so aufgebaut ist, dass die kontinuierlich im erfindungsgmässen Verhältnis getrennt zugeführten, intensiv vermischten Ausgangskomponenten bei einer bestimmten Temperatur so durch das Reaktionsgefäss geschleust werden, dass ein Vermischen von teilweise umgesetzten Ausgangsprodukten mit frisch zugeführten Ausgangskomponenten unmöglich ist. Erreicht wird dies im Prinzip dadurch, dass die Ausgangskomponenten durch Zusammenführen in einem bestimmten Mengenverhältnis in einer Mischkammer intensiv vermischt werden und

anschliessend unter Druck durch eine Autoklavenkaskade von mehreren — etwa 2-10 — Autoklaven bis zur völligen Umsetzung des Amins geleitet werden. Anschliessend erfolgt Entspannung und Auftrennung in dampfförmige und flüssige Anteile.

Anstelle der beschriebenen Autoklavenkaskade kann auch ein wesentlich einfacheres Reaktionsrohr eingesetzt werden, wobei die Zusammenführung der Reaktionskomponenten ebenfalls wieder in einer Mischkammer erfolgt. Das so entstandene Reaktionsgemisch wird anschliessend durch ein von aussen mit Dampf, Öl oder elektrisch beheiztes am zweckmässigsten spiralförmig angeordnetes Reaktionsrohr geführt, wobei die Länge so gross gewählt werden muss, dass eine bestimmte Verweildauer (von etwa 20 Minuten bei 150°C) bei vorgegebener Fliessgeschwindigkeit eingehalten wird. Anschliessend erfolgt ebenfalls Entspannung und Auftrennung in die verschiedenen Reaktionskomponenten.

Eine weitere Variante des Umsetzungsgefässes ist ein Schneckenreaktor, also ein von aussen beheiztes Rohr, in dessen Innerem eine Schnecke läuft, die das Rohr ganz ausfüllen kann, aber auch mit einem kleineren Durchmesser am Boden des Reaktionsgefässes sich befinden kann. Durch rotierende Bewegung der Schnecke wird das aus der Mischkammer in den Schneckenreaktor gelangende Reaktionsgemisch in der richtigen Reaktionszeit durch das Reaktionsgefäss geführt. Das Gefäss ist dabei so aufgebaut, dass ein Rückmischen unmöglich ist und dass durch entsprechende Vorrichtungen im Schneckengang ständig gute Durchmischung erreicht wird. Hiermit ist es praktisch möglich unter erhöhtem Druck wie auch bei Normaldruck die Umsetzung durchzuführen. Im Falle der Reaktionsführung bei Normaldruck setzt man einen Kühler auf und hält durch weitere geeignete Massnahmen den Schwefelkohlenstoff zurück.

Nach diesen allgemein beschriebenen Herstellungsverfahren werden sowohl wesentlich weniger Energie verbraucht als auch sehr viel weniger Arbeitszeit für die gleiche Menge hergestellter Substanz benötigt. Die Herstellungskosten liegen somit nach diesen Verfahren deutlich niedriger.

Produkte, die nach dem erfindungsgemässen Verfahren erhalten werden, weisen für einen Einsatz als Vulkanisationsbeschleuniger genügend hohe Reinheit auf. Sie lassen sich aber auch mit $H_2O$-Dampf strippen oder sogar destillieren. Kristalline Produkte können natürlich auch umkristalliert werden. Nebenprodukte die bei diesem Syntheseverfahren anfallen können, lassen sich durch einmaliges Waschen mit Wasser bei 80-90°C infolge Wasserlöslichkeit leicht entfernen. Niedrigschmelzende Thiazolidin-2-thione wie das 3-Methyl-thiazolidin-2-thion können hierbei in flüssigem Zustand durch Phasentrennung abgenommen und anschliessend auf einem Kristallisierband oder einer Schabewalze in eine in der Praxis gut verwertbare Handelsform gebracht werden.

Das bei diesem Verfahren nach dem Entspannen des Reaktionsgemisches von Thiazolidin-2-thion abgetrennte Abgasgemisch aus Schwefelwasserstoff und Kohlenoxysulfid enthält noch einen hohen Anteil an Schwefelkohlenstoff. Dieser Anteil wird durch Abkühlung des Gasgemisches auf <4°C auf etwa 20 Volumenprozent des Abgases reduziert. Restlicher Schwefelkohlenstoff kann durch eine Wäsche mit dem einzusetzenden Aminoalkohol vollständig aus dem Abgas entfernt werden. Dieses Gemisch aus Aminoalkohol und Dithiocarbamat kann dem Reaktor ebenfalls zugeführt werden, so dass der Schwefelkohlenstoff vollständig zurückgeführt wird.

Die Beseitigung der Reaktionsgase Schwefelwasserstoff und Kohlenoxydsulfid kann durch Absorption in Natronlauge oder durch Verbrennen und somit Überführung in Schwefelsäure erfolgen.

In den angegebenen Formeln bedeutet der Substituent $R_1$ bevorzugt einen gegebenenfalls substituierten Alkylrest mit 1 bis 18 C-Atomen, besonders bevorzugt mit 1 bis 8 C-Atomen oder einen Alkenylrest mit 3-18 C-Atomen, bevorzugt 3-8 C-Atomen. Als Substituenten des Alkyl- oder Alkenylrestes kommen beispielsweise in Betracht Mono- oder Dialkylamin, vorzugsweise mit $C_1$-$C_4$-Alkylgruppen, Alkoxyreste mit vorzugsweise $C_1$-$C_8$-Alkyl, Alkylthioreste mit vorzugsweise $C_1$-$C_4$-Alkyl, Halogen, vorzugsweise Chlor.

Beispielsweise seien folgende Reste aufgeführt:

Äthyl, Propyl, Isopropyl, Butyl, Isobutyl, sec.-Butyl, Methyl, tert.-Butyl, Pentyl, Isopentyl, sec.-Pentyl, tert.-Pentyl, Hexyl, sec.-Hexyl, tert.-Hexyl, Heptyl, Octyl, 2-Äthylhexyl, Nonyl, Decyl, Undecyl, Dodecyl, Tridecyl, Tetradecyl, Hexadecyl, Octadecyl, Dimethylaminoäthyl, Dimethylaminopropyl, Methoxyäthyl, Äthoxyäthyl, Isopropoxyäthyl, Propoxyäthyl, Methoxypropyl, Äthoxypropyl, Propoxypropyl, Isopropoxypropyl, Methylthioäthyl, Methylthiopropyl, Äthylthiopropyl, Äthylthioäthyl, Propylthioäthyl, Propylthiopropyl, Dimethylaminomethyl, Diäthylaminomethyl, Methoxymethyl, Äthoxymethyl, Propoxymethyl, Isopropoxymethyl, Butoxymethyl, Butylthiomethyl, Isopropylthiomethyl, Chloräthyl, Chlorpropyl, Cyclohexyl, Cyclopentyl, Cycloheptyl, Cyclohexylmethyl, Phenyl, 2-Methylphenyl, 3-Methylphenyl, 4-Methylphenyl, 2,3-Dimethylphenyl, 3,4-Dimethylphenyl, 4-Piperidinyl, 2,2,6,6-Tetramethylpiperidinyl, Morpholinyl, Phenylen, Äthylen, Propylen, Pentylen, Butylen, Hexylen, Isopropenyl, Phenyl-thioäthyl, Phenylthiopropyl, Äthylhexyloxypropyl, Diisobutyl, di-tert.-Dodecyl.

Sollte der Arylrest noch durch einen Alkylrest substituiert sein, kommen $C_1$-$C_4$-Alkylgruppen in Betracht.

Die Substituenten $R_2$ - $R_5$ stellen gleich oder

verschieden bevorzugt Wasserstoff oder Alkylgruppen mit 1 bis 6 C-Atomen dar.

Der Substituent $R_4$ bedeutet bevorzugt $C_2$-$C_6$-Alkylen.

Beispielhaft seien folgende Verbindungen genannt:

Thiazolidin-2-thion,
N-Methyl-thiazolidin-2-thion,
N-Äthyl-thiazolidin-2-thion,
N-Propyl-thiazolidin-2-thion,
N-Isopropyl-thiazolidin-2-thion,
N-Butyl-thiazolidin-2-thion,
N-sec.-Butyl-thiazolidin-2-thion,
N-tert.-Butyl-thiazolidin-2-thion,
N-Pentyl-thiazolidin-2-thion,
N-sec.Pentyl-thiazolidin-2-thion,
N-Isopentyl-thiazolidin-2-thion,
N-tert.Pentyl-thiazolidin-2-thion,
N-Isobutyl-thiazolidin-2-thion,
N-Hexyl-thiazolidin-2-thion,
N-sec.Hexyl-thiazolidin-2-thion,
N-Isohexyl-thiazolidin-2-thion,
N-tert.Hexyl-thiazolidin-2-thion,
N-Heptyl-thiazolidin-2-thion,
N-Isoheptyl-thiazolidin-2-thion,
N-tert.Heptyl-thiazolidin-2-thion,
N-Oktyl-thiazolidin-2-thion,
N-Isooktyl-thiazolidin-2-thion,
N-Nonyl-thiazolidin-2-thion,
N-Decyl-thiazolidin-2-thion,
N-Undecyl-thiazolidin-2-thion,

N-Dodecyl-thiazolidin-2-thion,
N-Tridecyl-thiazolidin-2-thion,
N-Tetradecyl-thiazolidin-2-thion,
N-Pentadecyl-thiazolidin-2-thion,
N-Hexadecyl-thiazolidin-2-thion,
N-Heptadecyl-thiazolidin-2-thion,
N-Oktadecyl-thiazolidin-2-thion,
N-Dimethylaminoäthyl-thiazolidin-2-thion,
N-Diäthylaminoäthyl-thiazolidin-2-thion,
N-Dipropylaminoäthyl-thiazolidin-2-thion,
N-Dimethylaminopropyl-thiazolidin-2-thion,
N-Diäthylaminopropyl-thiazolidin-2-thion,
N-Dipropylaminopropyl-thiazolidin-2-thion,
N-Dimethylaminomethyl-thiazolidin-2-thion,
N-Methoxyäthyl-thiazolidin-2-thion,
N-Äthoxyäthyl-thiazolidin-2-thion,
N-Propoxyäthyl-thiazolidin-2-thion,
N-Methoxypropyl-thiazolidin-2-thion,
N-Äthoxypropyl-thiazolidin-2-thion,
N-Propoxypropyl-thiazolidin-2-thion,
N-Diäthylaminomethyl-thiazolidin-2-thion,
N-Methylthiomethyl-thiazolidin-2-thion,
N-Äthylthiomethyl-thiazolidin-2-thion,
N-Propylthiomethyl-thiazolidin-2-thion,
N-Methylthioäthyl-thiazolidin-2-thion,
N-Äthylthioäthyl-thiazolidin-2-thion,
N-Propylthioäthyl-thiazolidin-2-thion,
N-Butylthioäthyl-thiazolidin-2-thion,
N-Methylthiopropyl-thiazolidin-2-thion,

N-Äthylthiopropyl-thiazolidin-2-thion,
N-Propylthiopropyl-thiazolidin-2-thion,
N-Butylthiopropyl-thiazolidin-2-thion,
N,N'-Propylen-bis-thiazolidin-2-thion,
N,N'-Butylen-bis-thiazolidin-2-thion,
N,N'-Pentylen-bis-thiazolidin-2-thion,
N,N'-Hexylen-bis-thiazolidin-2-thion,
N,N'-Phenylen-bis-thiazolidin-2-thion,
N-Phenyl-thioäthyl-thiazolidin-2-thion,
N-Phenyl-thiopropyl-thiazolidin-2-thion,
N-Hydroxyäthyl-thiazolidin-2-thion,
N-Hydroxypropyl-thiazolidin-2-thion,
N-Chloräthyl-thiazolidin-2-thion,
N-Chlorpropyl-thiazolidin-2-thion,
N-Cyclohexyl-thiazolidin-2-thion,
N-Cyclopentyl-thiazolidin-2-thion,
N-Cycloheptyl-thiazolidin-2-thion,
N-Cyclohexylmethyl-thiazolidin-2-thion,
N-Phenyl-thiazolidin-2-thion,
N-Methylphenyl-thiazolidin-2-thion,
N-Methyl-4,5-dimethyl-thiazolidin-2-thion,
N-Methyl-4,4,5-trimethyl-thiazolidin-2-thion,
N-Methyl-4,5,5-trimethyl-thiazolidin-2-thion,
N-Methyl-4,4-dimethyl-thiazolidin-2-thion,
N-Methyl-5,5-dimethyl-thiazolidin-2-thion,
N-Methyl-4,4,5,5-tetramethyl-thiazolidin-2-thion,
N-Äthyl-4,4,5,5-tetramethyl-thiazolidin-2-thion,
N-Äthyl-4,5-dimethyl-thiazolidin-2-thion,

N-Äthyl-4-methyl-thiazolidin-2-thion,
N-Äthyl-5-methyl-thiazolidin-2-thion,
N-Äthyl-4,4-dimethyl-thiazolidin-2-thion,
N-Äthyl-5,5-dimethyl-thiazolidin-2-thion,
N-Äthyl-4,5-diäthyl-thiazolidin-2-thion,
N-Äthyl-4-äthyl-thiazolidin-2-thion,
N-Äthyl-5-äthyl-thiazolidin-2-thion,
N-Äthyl-4-phenyl-thiazolidin-2-thion,
N-Methyl-4-äthyl-thiazolidin-2-thion,
N-Methyl-5-äthyl-thiazolidin-2-thion,
N-Cyclohexyl-4,5-dimethyl-thiazolidin-2-thion,
N-Cyclohexyl-4,4-dimethyl-thiazolidin-2-thion,
N-Cyclohexyl-5,5-dimethyl-thiazolidin-2-thion,
N-Cyclohexyl-5-äthyl-thiazolidin-2-thion,
N-Cyclohexyl-4-äthyl-thiazolidin-2-thion,
N-Phenyl-4,5-dimethyl-thiazolidin-2-thion,
N-Phenyl-5-äthyl-thiazolidin-2-thion,
N-(4-Piperidinyl-)-thiazolidin-2-thion,
N-[4-(2,2,6,6-Tetramethylpiperidinyl-)]-thiazolidin-2-thion,
N-Methyl-isopropenyl-thiazolidin-2-thion,
N-Phenyl-5-methyl-thiazolidin-2-thion,
N-Phenyl-4-phenyl-thiazolidin-2-thion,
N-Cyclohexyl-5-methyl-thiazolidin-2-thion,
N-Cyclohexyl-4-methyl-thiazolidin-2-thion,
N-Phenyl-5,5-dimethyl-thiazolidin-2-thion,
N-Phenyl-4,4-dimethyl-thiazolidin-2-thion,
N-Methyl-4,5-trimethylen-thiazolidin-2-thion,
N-Methyl-4,5-tetramethylen-thiazolidin-2-thion,
N-Methyl-4,5-pentamethylen-thiazolidin-2-thion,

N-Methyl-4,4,5,5-bis-trimethylen-thiazolidin-2-thion,
N-Methyl-4,4,5,5-bis-tetramethylen-thiazolidin-2-thion,
N-Methyl-4-methyl-thiazolidin-2-thion,
N-Methyl-5-methyl-thiazolidin-2-thion,
N-Äthylhexyl-thiazolidin-2-thion,

N-Methyl-4-phenyl-thiazolidin-2-thion,
N,N'-Äthylen-bis-thiazolidin-2-thion,
N,N'-Propylen-bis-thiazolidin-2-thion,
N-Isopropyl-5-methyl-thiazolidin-2-thion,
N-Isopropyl-4-methyl-thiazolidin-2-thion,
N-Isopropyl-4,5-dimethyl-thiazolidin-2-thion,
N-Isopropyl-5,5-dimethyl-thiazolidin-2-thion,
N-Isopropyl-4,4-dimethyl-thiazolidin-2-thion,
3,4-Trimethylen-thiazolidin-2-thion,
3,4-Tetramethylen-thiazolidin-2-thion,
3,5-Trimethylen-thiazolidin-2-thion,
3,5-Tetramethylen-thiazolidin-2-thion,
N-Methyl-4-vinyl-thiazolidin-2-thion,
N-Methyl-5-vinyl-thiazolidin-2-thion.

Die erfindungsgemäss hergestellten Verbindungen können als Vulkanisationsbeschleuniger für Polychloroprenkautschuke gemäss US-PS 3 215 703 eingesetzt werden.

Beispiel

3-Methylthiazolidin-2-thion lässt sich dadurch herstellen, dass man 2-Methylaminoalkohol und Schwefelkohlenstoff im molaren Verhältnis 1:4 in einer 150°C heissen Mischkammer mischt, wobei durch entsprechende Formgebung der Eintrittsdüsen und der Mischkammer sofort intensive Vermischung erfolgt. Hierbei stellt sich ein Druck von 50 bar ein. Nach Durchmischung erfolgt ein Transport in das eigentliche Reaktionsrohr. Nach 20 Minuten wird das Reaktionsgemisch in ein Entspannungsgefäss geführt und in gasförmige und flüssige Komponenten getrennt. Die hierbei ebenfalls abgekühlten Reaktionsgase durchlaufen erst einen Kühler, wo ein Teil des Schwefelkohlenstoffs flüssig abgeschieden wird, anschliessend den Schwefelkohlenstoffabsorber, der den Schwefelkohlenstoff fast vollständig dem Gasgemisch entnimmt. Noch vorhandener Schwefelwasserstoff und Kohlenoxydsulfid werden im $COS$-$H_2S$-Absorber völlig aufgenommen, was im Laborversuch durch Waschen mit Natronlauge, im technischen Massstab durch Verbrennen möglich ist.

Das im Entspannungsgefäss kondensierte rohe N-Methylthiazolidin-2-thion wird mit Wasserdampf andestilliert, um Reste von Schwefelkohlenstoff zu entfernen. Anschliessend kann aus Isobutanol umkristallisiert werden. Man erhält weisse Kristalle vom Schmp. 69-70°C. Natürlich kann auch das rohe Thiazolidin-2-thion kontinuierlich mit heissem Wasser (80-90°C) gewaschen, phasengetrennt einem Kristallisierband zugeführt und in eine praxisgerechte Form überführt werden.

Die einzusetzenden Aminoalkohole können auch mit einem zusätzlichen Lösungsmittel verdünnt in den Reaktor gepumpt werden. Insbesondere bei kristallinen Aminoalkoholen und höher schmelzenden Thiazolidin-2-thionen wird man diese Ausführungsform bevorzugen. Hierbei eignen sich natürlich nur inerte Lösungsmittel wie Kohlenwasserstoffe oder chlorierte Kohlenwasserstoffe, z. B. Cyclohexan, Benzol, Toluol, Xylol, Mesitylen, Cumol, Äthylbenzol, Chlorbenzol und Dichlorbenzol. Auch ätherartige Substanzen wie Diäthyläther, Diisopropyläther, Dioxan, Tetrahydrofuran sind verwendbar.

**Patentansprüche**

1. Verfahren zur Herstellung von Thiazolidin-2-thionen der allgemeinen Formel 1 und 2

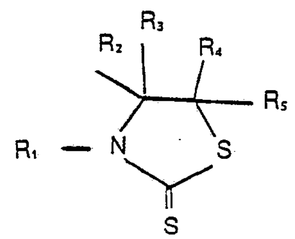

(1)

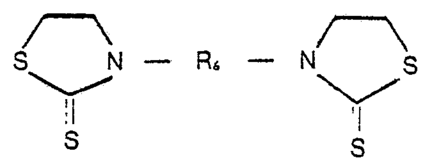

(2)

in denen

$R_1$ Wasserstoff oder einen geradkettigen, verzweigten oder cyclischen Alkylrest oder Alkenylrest, der gegebenenfalls durch Heteroatome O, S, N unterbrochen sein kann, oder einen Arylrest bedeutet, wobei die aufgeführten Reste gegebenenfalls auch durch Mono- oder Dialkylamino-, Alkoxy-, Alkylthio- und/oder Halogengruppen substituiert sein können und im Falle eines Arylrestes noch zusätzlich ein Alkylrest vorhanden sein kann,

$R_2$ und $R_3$ gleich oder verschieden Wasserstoff, einen Alkylrest oder Phenylrest bedeuten, wobei Alkylreste untereinander zu einem 5- oder 6gliedrigen Ring verbunden sein können,

$R_4$ und $R_5$ Wasserstoff einen Alkyl- oder Vinylrest und

$R_6$ eine Alkylen- oder Phenylengruppe darstellt,

durch Reaktion von entsprechenden Aminoalkoholen mit Schwefelkohlenstoff, dadurch gekennzeichnet, dass Schwefelkohlenstoff im molaren Mengenverhältnis zu Aminoalkohol von 4:1 bis 10:1 zugeführt und dass die Reaktion bei Temperaturen von 145-200°C und bei Normaldruck bis 100 bar in einstufiger Verfahrensweise durchgeführt wird.

2. Verfahren nach Anspruch 1, gekennzeichnet durch ein molares Mengenverhältnis von Schwefelkohlenstoff zu Aminoethanol von 4-6:1.

3. Verfahren nach Ansprüchen 1-2, dadurch gekennzeichnet, dass die Reaktion unter einem Druck von 20 bis 70 bar durchgeführt wird.

**Claims**

1. Process for the production of thiazolidine-2-thiones of the general formula 1 and 2

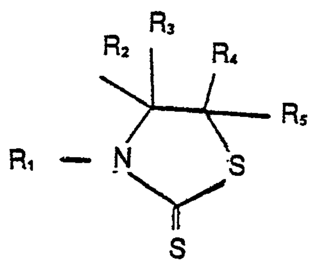

(1)

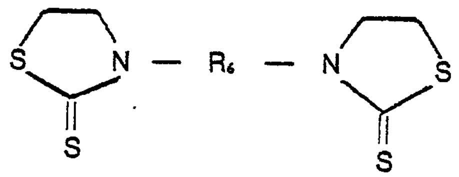

(2)

in which

$R_1$ denotes hydrogen or a straight chain, branched or cyclic alkyl radical or alkenyl radical, which can optionally be interrupted by the heteroatoms O, S or N, or an aryl radical, it being possible for the aforementioned radicals to be optionally substituted by monoalkylamino or dialkylamino, alkoxy, alkylthio and/or halogen groups and in the case of an aryl radical an alkyl radical can also additionally be present,

$R_2$ and $R_3$, which are identical or different, denote hydrogen, an alkyl radical or phenyl radical, it being possible for each alkyl radical to be contained in a 5-member or 6-member ring,

$R_4$ and $R_5$ represent hydrogen or an alkyl or vinyl radical and

$R_6$ represents an alkylene or phenylene group, by reacting corresponding amino alcohols with carbon disulphide, characterised in that carbon disulphide is introduced in a molar ratio to the amino alcohol of 4:1 to 10:1 and in that the reaction is carried out at temperatures of 145-200°C and at a pressure of from normal to 100 bar in a single-stage process.

2. Process according to Claim 1, characterised by a molar ratio of carbon disulphide to amino ethanol of 4-6:1.

3. Process according to Claims 1-2, characterised in that the reaction is carried out under a pressure of 20 to 70 bar.

**Revendications**

1. Procédé de production de thiazolidine-2-thiones de formules générales 1 et 2

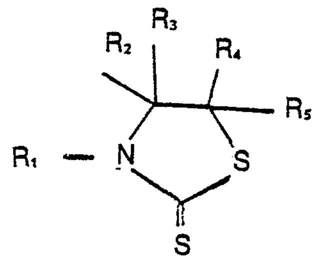

(1)

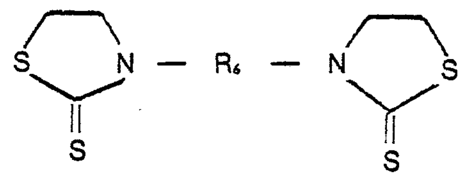

(2)

dans lesquelles:

$R_1$ est l'hydrogène ou un reste alkyle ou alcényle à chaîne droite, ramifié ou cyclique, qui est éventuellement interrompu par des hétéroatomes O, S, N ou désigne un reste aryle, les restes énumérés pouvant aussi être substitués, le cas échéant, par des groupes mono- ou dialkylamino, alkoxy, alkylthio et/ou halogéno et, dans le cas d'un reste aryle, un reste alkyle peut en outre être présent,

$R_2$ et $R_3$, égaux ou différents, désignent l'hydrogène, un reste alkyle ou le reste phényle, des restes alkyle pouvant être liés entre eux en un noyau pentagonal ou hexagonal,

$R_4$ et $R_5$ désignent l'hydrogène ou un reste alkyle ou vinyle et

$R_6$ désigne un groupe alkylène ou phénylène, par réaction d'amino-alcools correspondants avec le sulfure de carbone, caractérisé en ce qu'on fait arriver du sulfure de carbone dans un rapport de quantités molaires avec l'amino-alcool de 4:1 à 10:1 et en ce qu'on conduit la réaction à des températures de 145-200°C et à la pression normale et jusqu'à 100 bars en opérant en une seule étape.

2. Procédé suivant la revendication 1, caractérisé par un rapport des quantités molaires du sulfure de carbone à l'amino-éthanol de 4-6:1.

3. Procédé suivant les revendications 1-2, caractérisé en ce que la réaction est conduite à une pression de 20 à 70 bars.